# EUROPÄISCHE PATENTANMELDUNG

(11) **EP 2 644 175 A1**
(43) Veröffentlichungstag der Anmeldung: **02.10.2013**
(21) Anmeldenummer: 12161974.6
(22) Anmeldetag: 29.03.2012
(51) Int. Cl.: A61F 13/20, A61H 21/00, A61H 23/00

(54) **Tamponanordnung**

(71) Anmelder: Marlafin AG, 6332 Hagendorn (CH)
(72) Erfinder: Vaucher, Vincent, 2743 Eschert (CH); Aeschlimann, Marcel, 2514 Ligerz (CH); Wehren, Salome, 2503 Biel (CH)
(74) Vertreter: AMMANN PATENTANWÄLTE AG BERN

(57) **Zusammenfassung**

Die Tamponanordnung (1) umfasst einen Tampon (2) und einen darin angeordneten Vibrationserzeuger (3) sowie eine durch ein Verbindungselement (5) mit dem vibrationserzeuger (3) verbundenen, ausserhalb des Körpers der die Anordnung benutzenden Person zu tragenden Energiequelle (7). Das Verbindungselement (5) ist als flexibles Element zur Übertragung mechanischer Energie ausgebildet. Dadurch wird eine vibrierende Tamponanordnung zur Verfügung gestellt, bei der kein elektrischer Strom zum Tampon und damit in den Körper der die Tamponanordnung (1) benutzenden Person geleitet wird.

## Beschreibung

Die Erfindung betrifft eine Tamponanordnung mit einem in einem Tampon angeordneten Vibrationserzeuger und einer durch ein Verbindungselement mit dem Vibrationserzeuger verbundenen, ausserhalb des Körpers der die Tamponanordnung benutzenden Person anzuordnenden Energiequelle.

Vibrierende Tamponanordnungen sind bekannt, beispielsweise aus dem Dokument EP1231885B1. Diese Anordnung dient zur Bekämpfung von menstrualen Schmerzen und umfasst einen in einem Tampon angeordneten, elektrisch angetriebenen Vibrationserzeuger und eine mit dem Tampon durch ein Kabel verbundene, ausserhalb des Körpers zu tragende Energieversorgungseinheit, in welcher eine Energiequelle und ein als Drucktaste ausgebildetes Bedienungselement angeordnet ist, das die elektrische Verbindung zwischen der elektrischen Energiequelle und dem Vibrationserzeuger steuert. Wenn auch die zum Betrieb des Vibrationserzeugers verwendete Spannung relativ gering ist und beispielsweise 1,35 Volt beträgt und alle elektrischen Komponenten elektrisch isoliert sind, so haben doch viele potentielle Benutzerinnen Vorbehalte dagegen, elektrischen Strom in das Innere ihres Körpers zu leiten.

Ausgehend von diesem Stand der Technik liegt der Erfindung die Aufgabe zu Grunde, eine Tamponanordnung mit einem in einem Tampon angeordneten Vibrationserzeuger zur Verfügung zu stellen, bei der kein elektrischer Strom zum Tampon geleitet wird.

Diese Aufgabe wird erfindungsgemäss dadurch gelöst, dass das Verbindungselement als flexibles Element zur Übertragung mechanischer Energie ausgebildet ist.

Diese Lösung hat insbesondere den Vorteil, dass der vibrierende Tampon mit rein mechanischer Energie betrieben wird und insbesondere kein elektrischer Strom in den Körper der die Tamponanordnung benutzenden Person geleitet wird.

Nach einer Ausführungsart ist das flexible Element an seinem vom Tampon abgewandten Ende mit einem Bewegungsgenerator wirkverbunden. Letzterer ist vorteilhaft mit der Energiequelle in einem vorzugsweise gemeinsamen Gehäuse untergebracht.

Nach einer weiteren Ausführungsart ist das flexible Element als flexible Welle zur Übertragung eines Drehmoments ausgebildet. Flexible Wellen eignen sich gut zur Übertragung rein mechanischer Energie und sind mit kleinen Durchmessern, die sie für den Einsatz bei erfindungsgemässen Tamponanordnungen geeignet machen, im Handel erhältlich.

Gemäss einer weiteren Ausführungsart besteht das flexible Element aus einer Hülle und einem darin drehbaren Kernelement besteht. Flexible Wellen können ihren Zweck prinzipiell auch ohne Hülle erfüllen, jedoch ist es im vorliegenden Anwendungsfall vorteilhaft, eine Hülle zum Schutz der Umgebung zu verwenden.

Eine weitere Ausführungsart sieht vor, dass der Bewegungsgenerator so ausgebildet ist, dass ein mit ihm verbundener Abtriebsteil im Betrieb eine rotierende Bewegung ausführt. Diese rotierende Bewegung kann gleichförmig und gleichgerichtet sein, alternativ aber auch oszillierend, also abwechselnd um einen Winkelbetrag in eine Richtung und anschliessend in die entgegengesetzte Richtung. Durch diese Massnahmen erhöht sich die Freiheit bei der Gestaltung des im Tampon angeordneten Vibrationserzeugers.

Gemäss einer anderen Ausführungsart ist das flexible Element als Bowdenzug zur Übertragung von Zug- und/oder Druckkräften ausgebildet. Auch solche Elemente sind in für den Einsatz in einer Tamponanordnung geeigneten Dimensionen erhältlich. Der Einsatz eines solchen Elementes kann unter anderem auch bei der Gestaltung des Vibrationserzeugers vorteilhaft sein, indem dieser äusserst einfach aufgebaut werden kann.

Nach einer weiteren Ausführungsart ist der Bewegungsgenerator so ausgebildet, dass ein mit ihm verbundener Abtriebsteil im Betrieb eine oszillierende Translationsbewegung ausführt.

Schliesslich sieht noch eine Ausführungsart vor, dass das flexible Element mit dem Vibrationserzeuger und/oder mit dem Bewegungsgenerator durch eine Kupplung lösbar verbunden ist. Diese Massnahme erlaubt es, die ausserhalb des Körpers zu tragenden Teile der Tamponanordnung und gegebenenfalls auch das flexible Element mehrfach wieder zu verwenden, während der Tampon nach einmaligem Gebrauch entsorgt wird.

Gemäss einer Ausführungsform der Erfindung, welche sich durch besonders gute Eigenschaften beim Einsetzen und Ausführen des Tampons aus einer Körperöffnung auszeichnet, kann der Bewegungsgenerator so ausgebildet sein, dass ein mit ihm verbundener Abtriebsteil eine rotierende und eine Translationsbewegung ausführt.

Eine vorteilhafte Weiterbildung der Erfindung sieht vor, dass die rotierende und die Translationsbewegung einander synchron oder asynchron überlagert sind, wobei die Amplituden und/oder Frequenzen der einander überlagerten Bewegungen im Fall oszillierender Bewegungen unterschiedlich zueinander oder einander gleich sind.

Gemäss einer weiteren günstigen Variante der Erfindung kann der Bewegungsgenerator so ausgebildet sein, dass ein mit ihm verbundener Abtrieb zwischen einem Betrieb, in welchem er eine rotierende und eine Translationsbewegung ausführt, umschaltbar ist.

Eine Variante der Erfindung, welche sich besonders gut für die Aufnahme von Körperflüssigkeiten eignet, sieht vor, dass der den Vibrationserzeuger umgebende Tampon aus einem saugfähigen Material gebildet ist.

Entsprechend einer Ausführungsform der Erfindung, die sich durch eine besonders komfortable Bedienung auszeichnet und einen guten Schutz gegen Verschmutzungen von zur Wiederverwendung gedachten Teilen der Tamponanordnung bietet, kann es vorgesehen sein, dass der Vibrationserzeuger von dem Tampon umgeben, bevorzugt vollständig umgeben, ist und der mit der flexiblen Welle wirkverbundene Bewegungsgenerator vollständig ausserhalb des Tampons angeordnet ist. An dieser Stelle sei jedoch angemerkt, dass es mitunter auch von Vorteil sein kann, wenn der Vibrationserzeuger nur abschnittsweise von dem Tampon umgeben ist. Um ein ungewolltes Trennen von Tampon und Vibrationserzeuger zu vermeiden kann der Vibrationserzeuger zumindest ein Rückhalteelement aufweisen. Dieses Rückhaltelement kann beispielsweise durch zumindest einen Haken, zumindest eine Erhebung an der Oberfläche eines Gehäuses des Vibrationserzeugers oder durch zumindest eine Klebeschicht auf der Oberfläche eines Gehäuses Vibrationserzeuger oder eine gut an dem Tampon haftende Beschichtung eines Gehäuses des Vibrationserzeugers, etc. sein.

Die Erfindung samt weiteren Vorteilen wird im Folgenden anhand einiger nicht einschränkender Ausführungsbeispiele näher erläutert, welche in den zeichnungen dargestellt sind. In diesen zeigen jeweils in stark schematisch vereinfachter Darstellung:
- Figur 1: eine erste Ausführungsart einer Tamponanordnung;
- Figur 2: eine zweite Ausführungsart einer Tamponanordnung;
- Figuren 3 bis 6: Ausführungsarten eines Vibrationserzeugers der Tamponanordnungen aus Figur 1 und Figur 2;
- Figuren 7 bis 9: weitere Ausführungsarten eines Vibrationserzeugers;
- Figuren 10 bis 12: weitere Ausführungsarten eines Vibrationserzeugers;
- Figuren 13 bis 14: Ausführungsarten einer Kupplung der Tamponanordnungen aus Figur 1 und Figur 2;
- Figur 15: eine weitere Ausführungsart einer Tamponanordnung und
- Figur 16: eine perspektivische Ansicht der Tamponanordnung aus Figur 1.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäss auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäss auf die neue Lage zu übertragen. Weiter können auch Einzelmerkmale oder Merkmalskombinationen aus den gezeigten und beschriebenen unterschiedlichen Ausführungsbeispielen für sich eigenständige, erfinderische oder erfindungsgemässe Lösungen darstellen.

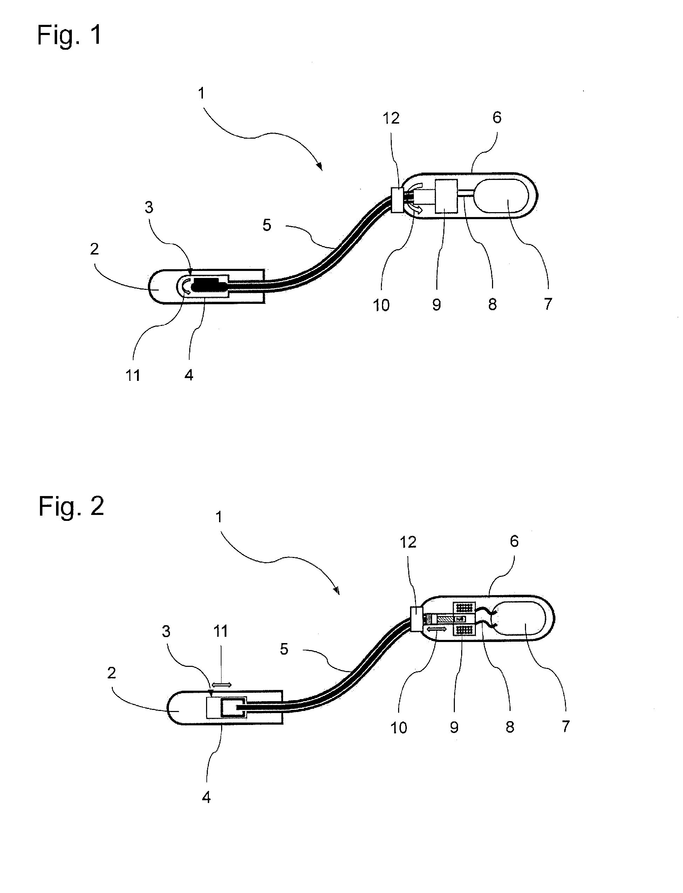

Die Figuren 1 und 2 zeigen zwei alternative Ausführungsarten der erfindungsgemässen Tamponanordnung 1. Ein Tampon 2 ist mit einem Vibrationserzeuger 3 ausgestattet, auf den weiter unten noch näher eingegangen wird. Der Vibrationserzeuger 3 ist in einem Gehäuse 4 untergebracht und durch ein Verbindungselement 5 mit einem weiteren Gehäuse 6 verbunden, das beim Tragen der Tamponanordnung 1 ausserhalb des Körpers verbleibt. Der Vibrationserzeugers 3 ist bevorzugt zur Gänze aus Kunststoff gefertigt. Insbesondere ist es von Vorteil, wenn zumindest das Gehäuse 4 des Vibrationserzeugers aus Kunststoff gefertigt ist. Auch das Verbindungselement 5 kann zur Gänze aus Kunststoff gefertigt sein. Durch die Ausbildung der oben genannten Komponenten aus Kunststoff, kann erreicht werden, dass keine metallischen Gegenstände in die Körperöffnung eingeführt werden müssen, was von manchen Personen als störend empfunden werden kann.

Im weiteren Gehäuse 6 befindet sich eine Energiequelle 7, die als elektrische Energiequelle wie Batterie oder Akkumulator, aber auch als beispielsweise pneumatische Energiequelle wie Druckgasspeicher ausgebildet sein kann. Die Energiequelle 7 ist mittels Leitungen 8 mit einem Bewegungsgenerator 9 verbunden, wobei in den Leitungen 8 vorteilhaft Steuermittel wie ein Schalter zwischengeschaltet sind, die in den Figuren nicht dargestellt sind. Mit 12 ist eine Kupplung bezeichnet, durch die das Verbindungselement 5 vom weiteren Gehäuse 6 trennbar ist. Durch die Kupplung 12 ist es möglich, das weitere Gehäuse 6 mit den darin angeordneten Komponenten wiederzuverwenden, während der Tampon 2 und das Verbindungselement 5 nach einmaliger Benutzung entsorgt werden. Alternativ ist es auch möglich, eine Kupplung beim Tampon 2 anzuordnen, so dass auch das Verbindungselement 5 wiederverwendbar ist. Diese Alternative ist in den Figuren nicht dargestellt.

Der Unterschied der Ausführungsarten nach Figur 1 und 2 besteht darin, dass bei der Ausführungsart nach Figur 1 der Bewegungsgenerator 9 eine rotierende Bewegung erzeugt, wie dies durch den Pfeil 10 dargestellt ist. Die rotierende Bewegung kann dabei kontinuierlich oder auch oszillierend sein, indem eine Welle des Bewegungsgenerators 9 durch den Bewegungsgenerator 9 um einen gewissen Betrag gedreht und anschliessend in der entgegengesetzten Richtung zurück gedreht wird. Das Verbindungselement 5 ist als flexible Welle ausgebildet, die vorteilhaft durch eine nicht rotierende Hülle geschützt ist. Derartige flexible wellen mit einem Durchmesser von beispielsweise 2 mm sind im Handel erhältlich uns werden beispielsweise im Modellbau oder in der Chirurgie eingesetzt. Die Drehbewegung des Bewegungsgenerators 9 wird über die flexible Welle 5 auf den Vibrationserzeuger 3 übertragen, wie dies durch den Pfeil 11 symbolisiert ist. Der Bewegungsgenerator 9 kann als Elektromotor, als Elektromagnet, als Pneumatikmotor, als Federmotor etc. ausgebildet sein. Im Gegensatz zur Ausführungsart nach Figur 1 wird bei der Ausführungsart nach Figur 2 durch den Bewegungsgenerator 9 eine hin- und hergehende Translationsbewegung erzeugt, welche durch eine als Bowdenzug ausgebildetes verbindungselement 5 zum Vibrationserzeuger 3 im Tampon 2 übertragen wird. Solche Bowdenzüge mit einer Hülle und einer darin axial verschiebbaren Seele sind ebenfalls in kleinen Durchmessern im Handel erhältlich und werden ebenfalls im Modellbau und in der Chirurgie eingesetzt, beispielsweise in einem Instrument gemäss DE19758596B4. Je nach Konstruktion und Materialwahl können die Seelen solcher Bowdenzüge auch Druckkräfte übertragen. Beispiele dafür sind die unter dem Handelsnamen "Flexball" erhältlichen Elemente. Der Betrieb ist aber auch mit reinen zugkräften möglich, indem an der dem Bewegungsgenerator gegenüberliegenden Seite des Verbindungselements 5 eine Zugfeder auf die Seele einwirkt. Der Bewegungsgenerator 9 kann beispielsweise ebenfalls als Elektromotor ausgebildet sein, dessen Drehbewegung durch Umwandlungsmittel wie eine Taumelscheibe oder einen kleinen Kurbeltrieb in eine Translationsbewegung umgewandelt wird. Ferner kann der Bewegungsgenerator 9 wie in Figur 2 schematisch dargestellt als Elektromagnet ausgebildet sein, oder alternativ als kleine pneumatische oder hydraulische Kolben-Zylinder-Einheit.

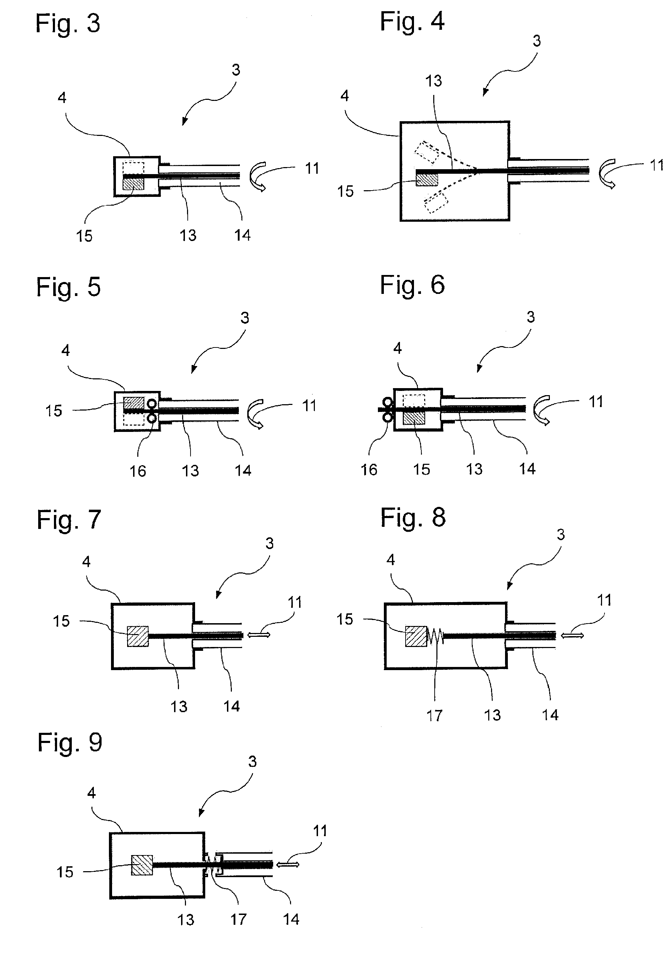

Die Figuren 3 bis 6 zeigen verschiedene Ausführungsarten des Vibrationserzeugers 3 für die Ausführungsart der Tamponanordnung 1 gemäss Figur 1 mit rotierendem Bewegungsgenerator 9 und flexibler Welle 5. Bei allen vier Ausführungsarten beruht das Prinzip der Vibrationserzeugung auf einer auf einer drehbaren Welle 13 exzentrisch gelagerten Masse 15. Dabei ist die drehende Welle 13 in einer nicht drehenden Hülle 14 angeordnet. Bei der Ausführungsart nach Figur 3 ist das in das Gehäuse 4 ragende, die Masse 15 tragende Ende der flexiblen Welle 13 relativ kurz. So bleibt das Wellenende im Betrieb relativ starr und die Masse 15 erzeugt die Vibration. Anders ist bei der Ausführungsart nach Figur 4 das in das Gehäuse 4 ragende, die Masse 15 tragende Ende der flexiblen Welle 13 relativ lang. Dies hat zur Folge, dass im Betrieb die rotierende Masse 15 das Wellenende verbiegt, wodurch die Masse 15 eine Kreisbahn beschreibt. Aus diesem Grund hat das Gehäuse 4 einen grösseren Durchmesser als das Gehäuse von Figur 3. Bei den Ausführungsarten nach den Figuren 5 und 6 ist das Ende der Welle 13 durch ein zusätzliches Lager 16 abgestützt, wobei das Lager 16 bei Figur 5 beim Eintritt der Welle 13 in das Gehäuse und bei Figur 6 auf der gegenüberliegenden Seite angeordnet ist. Selbstverständlich können auch beiderseits der Masse 15 Lager 16 angeordnet sein.

Die Figuren 7 bis 9 zeigen verschiedene Ausführungsarten des Vibrationserzeugers 3 für die Ausführungsart der Tamponanordnung 1 gemäss Figur 2 mit einem Bewegungsgenerator 9, der eine Translationsbewegung erzeugt und diese über eine Bowdenzug zum Tampon 2 überträgt. Zur Unterscheidung von der in den Figuren 3 bis 6 dargestellten rotierenden Welle 13 ist hier das in der Hülle 14 verschiebbare Element als Seele 18 bezeichnet. Die einfachste dieser Ausführungsarten ist in Figur 7 dargestellt. Die Masse 15 ist mit dem Ende der Seele 18 fest verbunden und erzeugt durch ihre Hin- und Her- Bewegung entsprechend dem Pfeil 11 die Vibration. Beim Ausführungsbeispiel nach Figur 8 ist die Masse 15 unter zwischenschaltung einer Feder 17 mit dem Ende der Seele 18 verbunden. Aufgrund des dadurch vergrösserten Weges, den die Masse 15 im Betrieb zurücklegt, ist das Gehäuse 4 etwas länger als das Gehäuse 4 von Figur 7. An dieser Stelle sei auch darauf hingewiesen, dass sowohl die Masse 15 als auch die Seele 18, so wie die anderen bei einer Benutzung in einer Körperöffnung aufgenommenen Teile des Vibrationserzeugers 3 aus Kunststoff gefertigt sein können. Bei der Ausführungsart nach Figur 9 ist eine Feder 17 zwischen der Hülle 14 und dem Gehäuse 4 angeordnet. Dadurch kann sich das Gehäuse 4 im Betrieb relativ zur Hülle 14 bewegen.

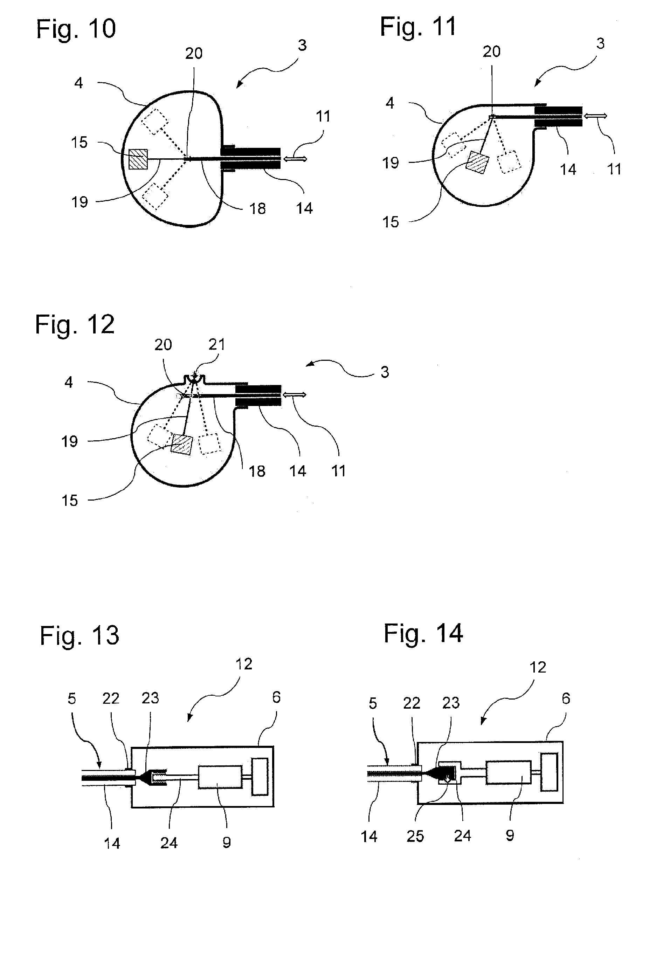

Drei weitere Ausführungsarten des vibrationserzeugers 3 für translatorische Bewegungen sind in den Figuren 10 bis 12 gezeigt. In einem Gehäuse 4 ist jeweils eine Masse 15 am freien Ende eines Hebels 19 angeordnet, der jeweils an einem Befestigungspunkt 20 mit dem freien Ende der Seele 18 verbunden ist. Bei der Ausführungsart nach Figur 10 kann die Masse 15 auf beide Seiten der Längsachse der Seele 18 schwingen, während bei der Ausführungsart nach Figur 11 die Masse 15 nur auf eine Seite der Längsachse der Seele 18 bewegbar ist. Bei beiden Ausführungsarten des Vibrationserzeugers nach den Figuren 10 und 11 entsteht im Betrieb auf Grund der frei schwingend gelagerten Masse 15 ein unregelmässiges Vibrationsmuster. Bei der Ausführungsart nach Figur 12 ist der Hebel 19 zusätzlich mittels eines Lagers 20 mit dem Gehäuse 4 verbunden, so dass sich hier im Betrieb ein regelmässiges Vibrationsmuster ergibt.

Die Figuren 13 und 14 zeigen zwei Ausführungsarten einer Kupplung 12, mit welcher das Verbindungselement 5 mit dem Bewegungsgenerator 9 lösbar verbunden ist. Wie weiter oben erwähnt, kann eine gleiche oder ähnliche Kupplung alternativ auch auf der Seite des Tampons 3 bzw. Vibrationserzeugers 3 angeordnet sein. Beide Kupplungen 12 sind mit kleinen Abwandlungen sowohl für rotatorische als auch für translatorische Bewegungen des Bewegungsgenerators 9 einsetzbar. Ein Kupplungsteil 22 für die Hülle 14 ist mit dem Gehäuse 6 verbunden und hat einen Innendurchmesser, der das axiale Passieren eines Kupplungsteils 23 der Welle 13 bzw. Seele 18 erlaubt. Mit dem Kupplungsteil 23 ist ein Abtriebsteil 24 des Bewegungsgenerators 9 lösbar verbunden. Für den Einsatz mit einem rotierenden Bewegungsgenerator 9 muss der Kupplungsteil 23 und der Abtriebsteil 24 des Bewegungsgenerators 9 so ineinander greifen, dass sie ein Drehmoment übertragen können. Umgekehrt müssen für den Einsatz mit einem Translationsbewegungen generierenden Bewegungsgenerator 9 der Kupplungsteil 23 und der Abtriebsteil 24 des Bewegungsgenerators 9 so ineinander greifen, dass sie eine linear gerichtete Kraft übertragen können. Als Beispiel ist in Figur 14 ein Rastelement 25 dargestellt.

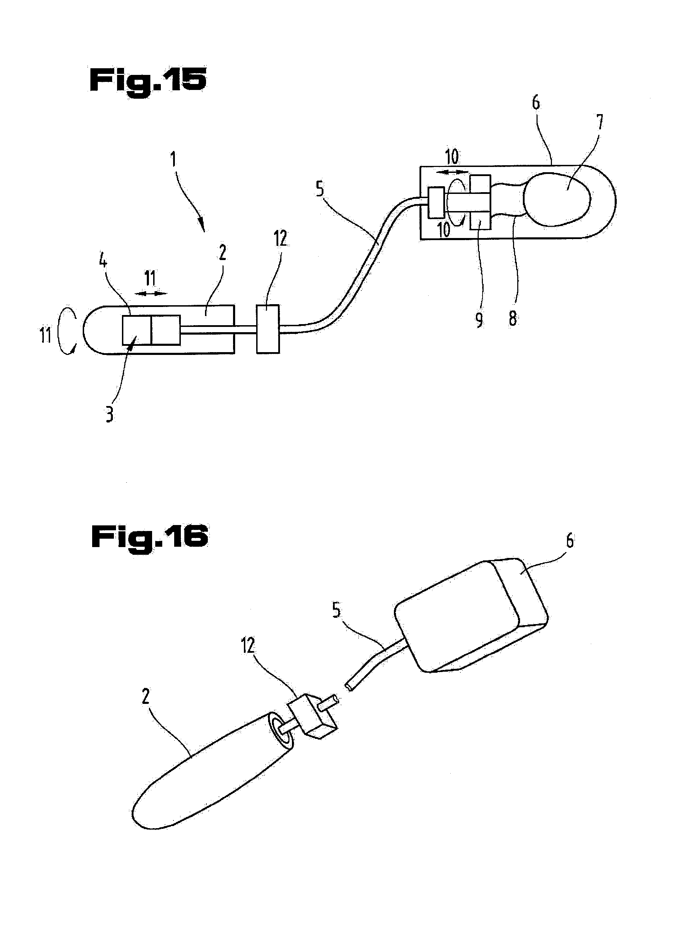

In der Fig. 15 ist eine weitere und gegebenenfalls für sich eigenständige Ausführungsform der erfindungsgemässen Tampoanordnung gezeigt, wobei wiederum für gleiche Teile gleiche Bezugszeichen bzw. Bauteilbezeichnungen wie in den vorangegangenen Figuren verwendet werden. Um unnötige Wiederholungen zu vermeiden, wird auf die detaillierte Beschreibung in den vorangegangenen Figuren hingewiesen bzw. Bezug genommen. Gemäss der dargestellten Ausführungsform kann der Vibrationserzeuger 3 eine Translations- und/oder eine Rotationsbewegung ausüben. Diese beiden Bewegungen können einander überlagert sein. Auch können bei diesem Ausführungsbeispiel sowohl die Translations- als auch die Rotationsbewegung gemeinsam oder getrennt aktivierbar sein. Jede dieser unterschiedlichen Translations- bzw. Rotationsbewegung kann getrennt oder gemeinsam abwechselnd aufeinanderfolgend oder zum Teil überlappend aktivierbar sein. Die Translations- und/oder die Rotationsbewegung können oszillierend oder intermittierend bzw. auch abwechselnd oszillierend oder intermittierend sein. Der Bewegungsgenerator 9 kann somit eine lineare Bewegung und eine Drehbewegung erzeugen, die auch einander überlagert und mittels des flexiblen Verbindungselements 5 auf den Vibrationserzeuger 3 übertragen werden können. Die Bewegungen der Translations-und Rotationsbewegungen sind in Fig. 15 mit den Bezugszeichen 10 und 11 versehen. Diese Ausführungsform ist vor allem hinsichtlich der Verbesserung der Ein- und Ausführbarkeit der aus Tampon 2 und Vibrationserzeuger 3 bestehenden Einheit in bzw. aus einer Körperöffnung von Vorteil. Eine Verbesserung der Ein- und/oder Ausführbarkeit der aus Tampon 2 und Vibrationserzeuger 3 bestehenden Einheit in bzw. aus einem Applikator kann dadurch ebenfalls erreicht werden. Hierzu ist es auch möglich die Frequenz und/oder die Amplitude der Translations- und/oder Rotationsbewegungen zu verändern z.B. die Amplitude der Translationsbewegung zu erhöhen.

Durch eine Überlagerung von Translations- und Rotationsbewegung des Vibrationserzeugers 3 lässt sich nämlich der Einführ- bzw. Ausführvorgang sehr effizient unterstützen. weiter kann es vorgesehen sein, dass zwischen der Erzeugung einer Rotationsbewegung und der Erzeugung einer Translationsbewegung manuell umgeschaltet werden kann. Dies kann beispielsweise durch Betätigung eines Schalters an dem Gehäuse 6 erfolgen. Darüber hinaus kann es vorgesehen sein, dass die Rotations- bzw. Translationsgeschwindigkeit bzw. die entsprechenden Beschleunigungen der von dem Bewegungsgenerator 9 erzeugten Bewegungen manuell eingestellt werden können. Dies kann auf unterschiedliche Weise realisiert sein, so kann der Bewegungsgenerator mit einer in dem Gehäuse 6 angeordneten Steuerung verbunden sein, beispielsweise einem programmierbaren Mikroprozessor, welcher mit einer Eingabeeinheit, beispielsweise einem Dreh/Schiebeschalter oder einem Touchscreen, auf dem Gehäuse 6 des Bewegungsgenerators verbunden ist. Die mit dem Bewegungsgenerator 9 verbundene Steuerung kann dann die entsprechende Steuerung der Bewegungsabläufe vornehmen.

Auch wäre es denkbar, dass der Bewegungsgenerator 9 über eine externe Steuerung, beispielsweise über ein Smartphone oder einen Tablet-Computer oder eine Fernbedienung, angesteuert wird. Hierzu kann der Bewegungsgenerator 9 über eine entsprechende Schnittstelle zur Kommunikation mit dem externen Gerät, drahtgebunden oder drahtungebunden, aufweisen. Die Einstellung des gewünschten Bewegungsablaufes kann dann von dem Benutzer über das Userinterface einer entsprechenden App oder Programms auf dem externen Gerät vorgenommen werden.

Wie in Fig. 15 dargestellt ist, kann die Kupplung 12 auch auf Seiten des Tampons 2 bzw. des Vibrationserzeugers 3 angeordnet sein. Die Kupplung 12 kann hierbei von dem Tampon etwas beanstandet sein, um einen hygienischen Wechsel der aus Tampon 2 und Vibrationserzeuger 3 bestehenden Einheit zu ermöglichen.

Wie aus Fig. 16 ersichtlich ist, kann der Vibrationserzeuger 3 von dem Tampon 2 umgeben sein. Der Tampon 2 als solcher ist perspektivisch dargestellt, wobei die flexible Verbindung 5 zu den im Inneren des Tampons 2 befindlichen Vibrationserzeugers 3 führt. Der Tampon 2 ist aus einem saugenden Material gefertigt. Im Folgenden soll kurz auf den Aufbau des Tampons 2 eingegangen werden. Der vorliegende Tampon besteht aus einem proximalen Ende und einem distalen Ende. Der Begriff "proximales Ende" 19, wie er hierin verwendet wird, bezieht sich auf jene Abschnitte der Anordnung und ihre Teile, die von dem Körper einer Benutzerin am weitesten entfernt sind, wenn der Tampon 2 in eine Körperöffnung, z.B. die Vagina, eingeführt wird. Der Begriff "distales Ende" bezieht sich auf jene Abschnitte der Anordnung und ihre Teile, die dem Körper einer Benutzerin am nächsten sind, wenn der Tampon eingeführt wird. Demzufolge geben die Begriffe "proximal" oder "distal", wie sie hierin verwendet werden, an, dass ein bestimmter Teil oder eine bestimmte Struktur der Anordnung oder ihre Teile dem proximalen Ende bzw. dem distalen Ende der Anordnung oder ihrer Teile näher ist. In ähnlicher Art und Weise beziehen sich die Begriffe "proximale Richtung" oder "distale Richtung" auf die Richtungen zum proximalen Ende bzw. zum distalen Ende des Tampons 2 hin.

Der Tampon 2 besitzt einen länglichen Hauptteil, der ein distales Einschubende, ein proximales Rückzugsende und einen mittleren Teil bildet, der dazwischen verläuft. Der Tampon besitzt ausserdem eine Längsachse und eine Aussenseite aus einem saugenden Material. Der Tampon 2 oder zumindest der mittlere Teil des Tampons 2 kann eine im Wesentlichen zylindrische Form aufweisen, wobei. es sich um eine einfache geometrische Form einer zylindrischen Hülle handeln kann, die der Gesamtform des Tampons 2 oder zumindest ihres mittleren Teils am nächsten kommt. Der Tampon 2 kann an seiner Aussenseite auch Rippen und Rillen aufweisen oder an seiner Aussenseite im Wesentlichen glatt ausgebildet sein. Der maximale Aussendurchmesser des Tampon s 2 oder seines mittleren Abschnittes kann in Längsrichtung im Wesentlichen. gleichmässig sein oder er kann variieren. Der Teil des Tampons 2 nahe an dem Rückzugsende, an welchem die flexible Verbindung 5 angeordnet ist, kann beispielsweise einen grösseren maximalen Aussendurchmesser besitzen als der restliche Teil des mittleren Abschnittes. Durch dieses Dickerwerden am proximalen Ende des Tampons 2 verringert sich die Gefahr, dass Körperflüssigkeit austritt, wenn der Tampon 2 in der Vagina platziert wird.

Zur Herstellung der Einheit aus Vibrationserzeuger 3 und Tampon 2 kann der Vibrationserzeuger 3 mit einem saugfähigen Fasermaterial umwickelt bzw. umgeben werden, welches den Tampon 2 bildet. Der Vibrationserzeuger 3 kann beispielsweise in das Material des Tampons 2 eingenäht werden oder aber auch durch eingangs genannte Rückhaltelemente an einem Herausrutschen aus dem Tampon gehindert werden. Alternativ sind jedoch noch eine grosse Anzahl anderer Verfahren zur Verbindung des Vibrationserzeugers 3 mit dem Tampon 2 möglich, so könnte nach einem Umwickeln des Vibrationserzeugers mit dem Tampon 2 das distale Ende des Tampons 2 dicht gepresst werden, sodass dieser gepresste Abschnitt ein Herausziehen des Vibrationserzeugers verhindert.

Saugfähiges Fasermaterial für den Tampon 2 kann aus jedem beliebigen, saugfähigen Material bestehen, das akzeptable saugfähigkeitseigenschaft und Elastizitätsmoduleigenschaften besitzt, welche Flüssigkeit aufnehmen und/oder zurückhalten können. Die saugfähige Struktur lässt sich aus einer Vielzahl von Grössen und Formen und aus einer Vielzahl von flüssigkeitsabsorbierenden Materialien herstellen. Selbstverständlich ist es wünschenswert, saugfähige Materialien zu verwenden, die einen Mindestgehalt an fremdlöslichen Materialien enthalten, da das Produkt für einen bestimmten Zeitraum im Körper verbleibt. Zurückgehaltene lösliche Fremdmaterialien könnten ein Sicherheitsrisiko darstellen, wenn sie toxisch, reizend oder empfindlich sind.

Eine repräsentative, nicht einschränkend zu verstehende Liste brauchbarer Materialien enthält zellulosehaltige Materialien wie beispielsweise Rayon, Baumwolle, Zellstoff, Zellstoffwatte, Tissue, Tissue-Schichstoffe, Torfmull und chemisch verstärkte, modifizierte oder vernetzte Zellulosefasern; synthetische Materialien wie beispielsweise Polyesterfasern, Polyolefinfasern, saugfähige Schaumstoffe, beispielsweise ein elastisch federnder Polyurethanschaumstoff, saugfähige Schwämme äusserst saugfähige Polymere, saugfähige, gelbildende Materialien; bearbeitete Fasern wie beispielsweise kapillare Kanalfasern und Fasern mit mehreren Gliedern; synthetische Fasern oder ein äquivalentes Material oder Kombinationen von Materialien oder Mischungen daraus.

Die einzelnen in den Figuren gezeigten Ausführungen können auch den Gegenstand von eigenständigen, erfindungsgemässen Lösungen bilden. Die diesbezüglichen, erfindungsgemässen Aufgaben und Lösungen sind den Detailbeschreibungen dieser Figuren zu entnehmen. Der Ordnung halber sei darauf hingewiesen, dass zum besseren Verständnis des Aufbaus des erfindungsgemässen Endlosbandes dieses bzw. dessen Bestandteile teilweise unmassstäblich und/oder vergrössert und/oder verkleinert dargestellt wurden.

Die Ausführungsbeispiele zeigen mögliche Ausführungsvarianten der erfindungsgemässen Tamponanordnung, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mitumfasst.

### Bezugszeichenliste

- 1: Tamponanordnung
- 2: Tampon
- 3: Vibrationserzeuger
- 4: Gehäuse
- 5: Verbindungselement
- 6: Gehäuse
- 7: Energiequelle
- 8: Leitungen
- 9: Bewegungsgenerator
- 10: Pfeil
- 11: Pfeil
- 12: Kupplung
- 13: Welle
- 14: Hülle
- 15: Masse
- 16: Lager
- 17: Feder
- 18: Seele
- 19: Hebel
- 20: Befestigungspunkt
- 21: Lager
- 22: Kupplungsteil für Hülle
- 23: Kupplungsteil von Welle bzw. Seele
- 24: Abtriebsteil
- 25: Rastelement

## Patentansprüche

1. Tamponanordnung (1) mit einem in einem Tampon (2) angeordneten Vibrationserzeuger (3) und einer durch ein Verbindungselement (5) mit dem Vibrationserzeuger (3) verbundenen, ausserhalb des Körpers der die Tamponanordnung benutzenden Person anzuordnenden Energiequelle (7), **dadurch gekennzeichnet, dass** das Verbindungselement (5) als flexibles Element zur Übertragung mechanischer Energie ausgebildet ist.

2. Tamponanordnung (1) nach Anspruch 1, **dadurch gekennzeichnet, dass** das flexible Element (5) an seinem vom Tampon (2) abgewandten Ende mit einem Bewegungsgenerator (9) wirkverbunden ist.

3. Tamponanordnung (1) nach Anspruch 2, **dadurch gekennzeichnet, dass** der Bewegungsgenerator (9) und die Energiequelle (7) in einem vorzugsweise gemeinsamen Gehäuse (6) angeordnet sind.

4. Tamponanordnung (1) nach einem der vorangehenden Ansprüche, **dadurch gekennzeichnet, dass** das flexible Element (5) als flexible Welle zur Übertragung eines Drehmoments ausgebildet ist.

5. Tamponanordnung nach Anspruch 4, **dadurch gekennzeichnet, dass** das flexible Element (5) aus einer Hülle (14) und einem darin drehbaren Kernelement (13) besteht.

6. Tamponanordnung (1) nach einem der vorangehenden Ansprüche, **dadurch gekennzeichnet, dass** der Bewegungsgenerator (9) so ausgebildet ist, dass ein mit ihm verbundener Abtriebsteil (24) im Betrieb eine rotierende Bewegung ausführt.

7. Tamponanordnung (1) nach Anspruch 6, **dadurch gekennzeichnet, dass** der Bewegungsgenerator (9) so ausgebildet ist, dass die rotierende Bewegung oszillierend ist.

8. Tamponanordnung (1) nach einem der Ansprüche 1 bis 2, **dadurch gekennzeichnet, dass** das flexible Element (5) als Bowdenzug zur Übertragung von Zug- und/oder Druckkräften ausgebildet ist.

9. Tamponanordnung (1) nach einem der Ansprüche 1 bis 2 und 8, **dadurch gekennzeichnet, dass** der Bewegungsgenerator so ausgebildet ist, dass ein mit ihm verbundener Abtriebsteil (24) im Betrieb eine oszillierende Translationsbewegung ausführt.

10. Tamponanordnung (1) nach einem der vorangehenden Ansprüche, **dadurch gekennzeichnet, dass** das flexible Element (5) mit dem Vibrationserzeuger (3) und/oder mit dem Bewegungsgenerator (9) durch eine Kupplung (12) lösbar verbunden ist.

11. Tamponanordnung nach einem der vorhergehenden Ansprüche, **dadurch gekennzeichnet, dass** der Bewegungsgenerator (9) so ausgebildet ist, dass ein mit ihm verbundener Abtriebsteil (21) eine rotierende und eine Translationsbewegung ausführt.

12. Tamponanordnung nach Anspruch 11,**dadurch gekennzeichnet, dass** die rotierende und die Translationsbewegung einander synchron oder asynchron überlagert sind, wobei die Amplituden und/oder Frequenzen der einander überlagerten Bewegungen im Fall oszillierender Bewegungen unterschiedlich zueinander oder einander gleich sind.

13. Tamponanordnung nach einem der vorhergehenden Ansprüche, **dadurch gekennzeichnet, dass** der Bewegungsgenerator so ausgebildet ist, dass ein mit ihm verbundener Abtrieb zwischen einem Betrieb, in welchem er eine rotierende und eine Translationsbewegung ausführt, umschaltbar ist.

14. Tamponanordnung nach einem der vorhergehenden Ansprüche, **dadurch gekennzeichnet, dass** der den Vibrationserzeuger (3) umgebende Tampon (2) aus einem saugfähigen Material gebildet ist.

15. Tamponanordnung nach Anspruch 14, **dadurch gekennzeichnet, dass** der Vibrationserzeuger (3) von dem Tampon (2) umgeben, bevorzugt vollständig umgeben, ist und der mit der flexiblen Welle wirkverbundene Bewegungsgenerator (9) vollständig außerhalb des Tampons (2) angeordnet ist.
